# EUROPEAN PATENT APPLICATION

(11) **EP 4 834 957 A1**
(43) Date of publication of application: **04.11.2026**
(21) Application number: 25774051.4
(22) Date of filing: 18.03.2025
(51) Int. Cl.: C08F 36/20, C07D 209/76, C08F 222/40, C08F 236/20, C08L 101/00

(54) **BISALKENYL-SUBSTITUTED NADIMIDE, METHOD FOR PRODUCING SAME, CURABLE MATERIAL, CURABLE RESIN COMPOSITION, AND CURED PRODUCT OF SAME**

(30) Priority: 21.03.2024 JP 2024045579
(71) Applicant: Maruzen Petrochemical Co., Ltd., Tokyo 104-8502 (JP)
(72) Inventor: ODA Takashi, Ichihara-shi, Chiba 290-8503 (JP)
(74) Representative: Regimbeau
(86) International application number: PCT/JP2025/010424
(87) International publication number: WO 2025/197900

(57) **Abstract**

[Problem] Provided is a bisalkenyl-substituted nadimide that has a low dielectric constant and a low dielectric loss factor in the GHz band (particularly in the 10 GHz band), and yields a film having excellent flexibility.

[Solution] A bisalkenyl-substituted nadimide according to the present invention is shown by the following General Formula (1): 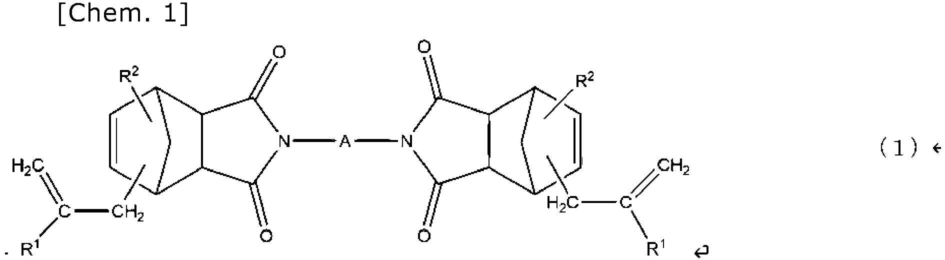 wherein, in Formula (1), R¹ and R² are each a hydrogen atom or a C₁-C₆ alkyl group selected independently; and A is a divalent hydrocarbon group derived from a dimer acid skeleton.

## Description

### BACKGROUND OF THE INVENTION

### Field of the Invention

The present invention relates to a bisalkenyl-substituted nadimide, a method of producing the same, a curable material, a curable resin composition, and a cured product of the material or the composition.

### Background Art

Along with the advancement of science and technology, materials to be used are desired to have higher performance and higher functionality, and various materials have been developed to meet these demands. For example, various high-performance polymers called super engineering plastics (engineering plastics), such as polyether ketone, polyphenylene sulfide, and polyimide, are widely available in the market. Among others, polyimides are very promising in the future from the viewpoints of heat resistance and the like.

Among polyimides, bismaleimides having imide skeletons at both ends are often used. A bismaleimide resin has a rigid imide ring, and thus, exhibits higher heat resistance and lower thermal expandability than an epoxy resin. In addition, a bismaleimide generates no volatile gas during molding, and thus, is used mainly in the fields of aerospace, electronic materials, and the like.

Furthermore, among these, a bisnadimide, which is a bismaleimide having norbornene rings at both ends, has a higher working-temperature upper limit than a bismaleimide having no norbornene ring at both ends. Accordingly, bisnadimides have conventionally attracted attention as addition-polyimide resin raw materials having extremely high heat resistance. Some of the bisnadimides have been put into practical use as what is called a matrix for an advanced composite material. However, such bisnadimides generally have a high melting point and poor solubility in a solvent, and thus, are difficult to handle. In addition, the bisnadimide lacks reactivity, and thus, involves stringent reaction conditions (for example, high-temperature molding such as performed at 300°C) in order to have a higher molecular weight.

Under such reaction conditions, however, not only a higher molecular weight reaction of interest but also the retro-Diels-Alder reaction of the norbornene ring partly occurs. It is said that highly volatile cyclopentadiene generated under such conditions becomes a cause of generation of considerable voids in a molded product.

Examples of a molding method intended to inhibit such generation of voids include: a method in which reaction is caused at high temperature under pressure (for example, an autoclave molding method); and a method in which an acid anhydride or an ester and a diamine, as a raw material for an imide, are dissolved in a solvent to be formed into an oligomer, which is used in varnish form. However, these forms of usage have room for improvement from the viewpoints of involving high-temperature pressurized conditions and the volatilization of a solvent.

On the other hand, to inhibit this generation of voids, various methods other than adopting the above-described means have been studied, as follows: a method in which the curing reaction temperature is decreased; and a method in which a suitable substituent is introduced into the above-described norbornene ring so that no volatile component can be generated even if the retro-Diels-Alder reaction has occurred.

Patent Literature 1 to 3 discloses various inventions related to a bisalkenyl-substituted nadimide. Patent Literature 1 and Patent Literature 2 and 3 disclose known bisalkenyl-substituted nadimides such as bis{4-(allylbicyclo[2.2.1]hepto-5-ene-2,3-dicarboxyimide)phenyl}methane and N,N'-m-xylylene-bis(allylbicyclo[2.2.1]hepto-5-ene-2,3-dicarboxy imide respectively.

In such a manner, the above-described bisalkenyl-substituted nadimide is characterized by having excellent mechanical strength, heat resistance up to 300°C, and dimensional stability, and used as the following: a heat-resistant adhesive; a precoat agent for metal adhesion of TEFLON; a heat resistance improver for an epoxy resin; a solder agent; a carbon fiber sizing agent; a material for a printed board; or the like.

### Citation List

### Patent Literature

[Patent Literature 1] JPS61-73710A
[Patent Literature 2] JPH07-53516A
[Patent Literature 3] JPH07-224121A

### SUMMARY OF THE INVENTION

### Technical Problem

However, a cured product of the bisalkenyl-substituted nadimide, owing to its hardness and brittleness, is unable to be widely used in the fields such as a film in need of flexibility, and is difficult to use for a thin membrane or a film. In addition, the nadimide is solid or semisolid, thus needs heating to obtain fluidity, and will undesirably be restricted in being blended with another resin. Furthermore, the dielectric characteristics of the nadimide, for example, the dielectric constant and dielectric loss factor in the GHz band, particularly in the high-frequency band of 10 GHz or more, are unsatisfactory for the 5G era from a properties viewpoint.

In view of this, an object of the present invention is to provide a bisalkenyl-substituted nadimide that has a low dielectric constant and a low dielectric loss factor in the GHz band, particularly in the high-frequency band of 10 GHz or more, and yields a film having excellent flexibility.

### Solution to Problem

The present inventors have vigorously studied to achieve the above-described object, and have come to complete the present invention through the discovery that the above-described problems can be solved by synthesizing a novel bisalkenyl-substituted nadimide having a specific structure.

That is, the present invention provides the following inventions.
[1] A bisalkenyl-substituted nadimide of General Formula (1): 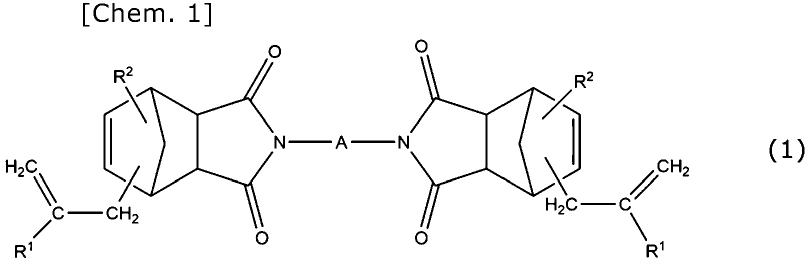
   wherein, in Formula (1), R¹ and R² are each a hydrogen atom or a C₁-C₆ alkyl group selected independently; and
   A is a divalent hydrocarbon group derived from a dimer acid skeleton.
[2] The bisalkenyl-substituted nadimide according to [1], wherein the divalent hydrocarbon group derived from the dimer acid skeleton comprises a structure of General Formula (2): 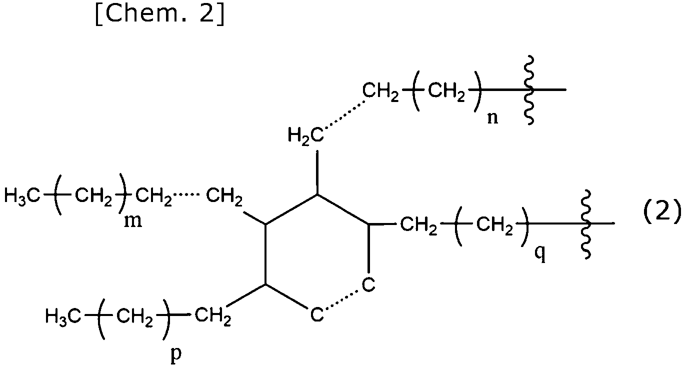 wherein, in Formula (2), m, n, p, and q are each an integer of 1 or greater selected so as to satisfy m + n = 5 to 16 and p + q = 8 to 19; and the bonds shown by the dashed lines are each independently a carbon-carbon single bond or a carbon-carbon double bond, with the proviso that, in a case where the bond shown by each dashed line is a carbon-carbon double bond, Formula (2) is a structure in which the number of hydrogen atoms bound to each of the carbon atoms constituting the carbon-carbon double bond is determined by subtracting 1 from each number shown by Formula (2); and the wavy lines are each a binding site to the nitrogen atom of an imide.
[3] A method of producing the bisalkenyl-substituted nadimide according to [1] or [2], comprising subjecting a dimer diamine and a compound of General Formula (3) to an addition-dehydration reaction; 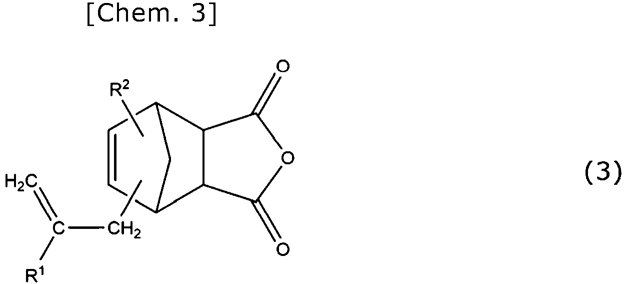 wherein, in Formula (3), R¹ and R² are each a hydrogen atom or a C₁-C₆ alkyl group selected independently.
[4] The method of producing the alkenyl-substituted nadimide according to [3], wherein the dimer diamine comprises a structure of General Formula (4): 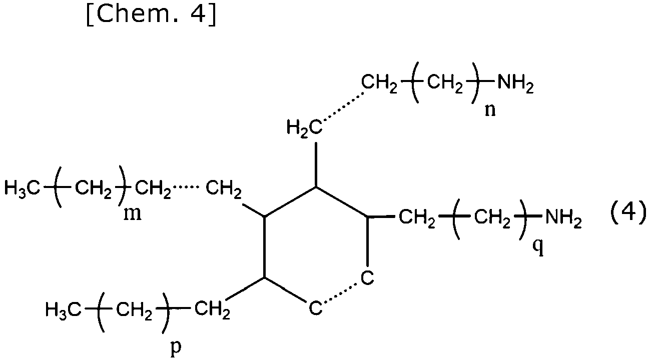 wherein, in Formula (4), m, n, p, and q are each an integer of 1 or greater selected so as to satisfy m + n = 5 to 16 and p + q = 8 to 19; and the bonds shown by the dashed lines are each independently a carbon-carbon single bond or a carbon-carbon double bond, with the proviso that, in a case where the bond shown by each dashed line is a carbon-carbon double bond, Formula (4) is a structure in which the number of hydrogen atoms bound to each of the carbon atoms constituting the carbon-carbon double bond is determined by subtracting 1 from each number shown by Formula (4).
[5] A curable material comprising the bisalkenyl-substituted nadimide according to [1] or [2].
[6] A curable resin composition comprising:
   (A) the bisalkenyl-substituted nadimide according to [1] or [2]; and
   (B) at least one compound selected from the group consisting of a maleimide compound, an alkenyl-substituted nadimide (excluding the bisalkenyl-substituted nadimide according to [1] or [2]), an epoxy resin, a phenol resin, a vinylbenzyl compound, a vinyl compound (excluding a vinylbenzyl compound), a cyclic olefin, a conjugated diene resin having a functional group, and an unsaturated polyester resin.
[7] The curable resin composition according to [6], wherein the maleimide compound is a bismaleimide of General Formula (5): 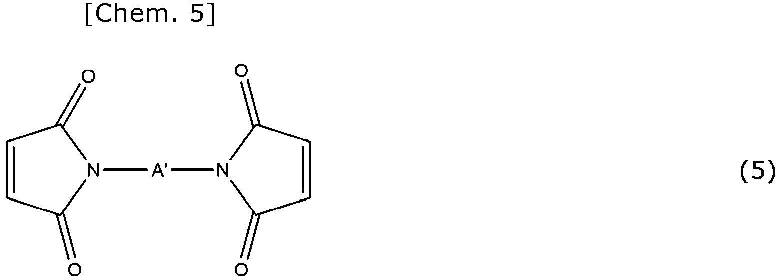 wherein, in Formula (5), A' is a divalent hydrocarbon group.
[8] The curable resin composition according to [7], wherein A' in the General Formula (5) is a divalent hydrocarbon group derived from a dimer acid skeleton.
[9] The curable resin composition according to any one of [6] to [8], wherein the mass ratio (A)/(B) of the (A) bisalkenyl-substituted nadimide according to [1] or [2] to the (B) is in the range of from 90/10 to 10/90.
[10] The curable material according to [5], used for an electronic material.
[11] The curable resin composition according to any one of [6] to [9], used for an electronic material.
[12] A cured product obtained by curing the curable material according to [5] or [10].
[13] A cured product obtained by curing the curable resin composition according to any one of [6] to [9] and [11].

### Advantageous Effects of Invention

The present invention can provide: a bisalkenyl-substituted nadimide that has a low dielectric constant and a low dielectric loss factor in the GHz band, particularly in the high-frequency band of 10 GHz or more, and yields a film having excellent flexibility; and a method of producing the bisalkenyl-substituted nadimide. Accordingly, a curable material or curable resin composition comprising a bisalkenyl-substituted nadimide according to the present invention can be suitably used as a film for an electronic material satisfactory for 5G.

### BRIEF DESCRIPTION OF THE DRAWINGS

FIG. 1 illustrates the IR spectrum of the bisalkenyl-substituted nadimide A synthesized in Example 1.
FIG. 2 illustrates the ¹H-NMR spectrum of the bisalkenyl-substituted nadimide A synthesized in Example 1.
FIG. 3 illustrates the ¹³C-NMR spectrum of the bisalkenyl-substituted nadimide A synthesized in Example 1.

### DETAILED DESCRIPTION OF THE INVENTION

### (Bisalkenyl-substituted nadimide)

A bisalkenyl-substituted nadimide according to the present invention is a compound having a structure of the following General Formula (1):

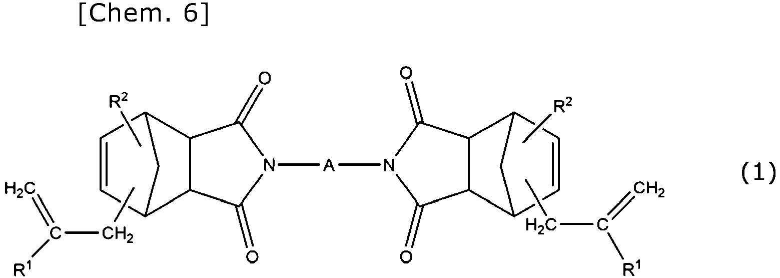

In Formula (1) above, R¹ and R² are each a hydrogen atom or a C₁-C₆ alkyl group selected independently, preferably each a hydrogen atom or a C₁-C₃ alkyl group selected independently, more preferably each a hydrogen atom or a methyl group selected independently, still more preferably each a hydrogen atom.

In Formula (1), A is a divalent hydrocarbon group derived from a dimer acid skeleton. A dimer acid is a dibasic acid of a dicarboxylic acid generated by dimerizing an unsaturated fatty acid derived from the raw material of which is a natural product such as plant fat. Dimer acids exist, not having a single structure but having a plurality of structures or isomers.

Specific examples of the structure comprise, but are not limited to, the following.

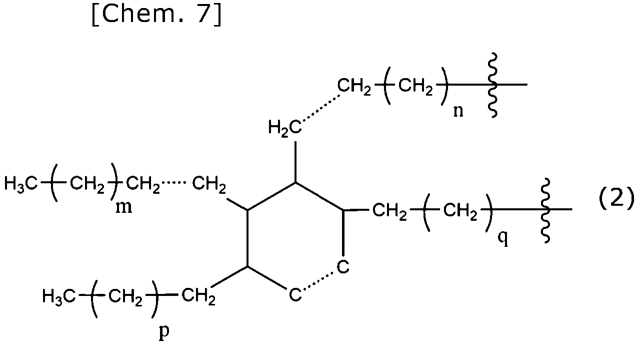

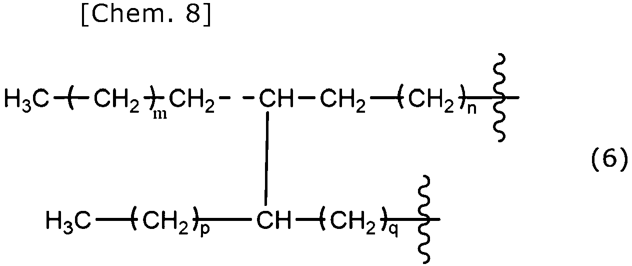

In Formulas (2) and (6), m, n, p, and q are each an integer of 1 or greater selected so as to satisfy m + n = 5 to 16 and p + q = 8 to 19; and the bonds shown by the dashed lines each mean a carbon-carbon single bond or a carbon-carbon double bond, with the proviso that, in a case where the bond shown by each dashed line is a carbon-carbon double bond, Formulas (2) and (6) are each a structure in which the number of hydrogen atoms bound to each of the carbon atoms constituting the carbon-carbon double bond is determined by subtracting 1 from each number shown in Formulas (2) and (6). The wavy lines are each a binding site to the nitrogen atom of an imide. In a preferable aspect of the present invention, A comprises a structure of Formula (2).

A bisalkenyl-substituted nadimide according to the present invention is liquid at room temperature, and thus, is easy to disperse with another component to prepare a curable resin composition. In addition, when cured, a conventionally known bisalkenyl-substituted nadimide having a structure comprising an aromatic ring yields a hard and brittle cured product, but in contrast, a cured product of a bisalkenyl-substituted nadimide according to the present invention has flexibility, and can be molded in film form. This is considered to be because, in a case where A in Formula (1) comprises a ring structure, the bisalkenyl-substituted nadimide can take a larger volume, thus achieving liquidness, and allowing the resultant cured product to have excellent flexibility. This is merely the present inventors' inference, and the present invention should not be bound by this theory.

### [Method of producing bisalkenyl-substituted nadimide]

A bisalkenyl-substituted nadimide according to the present invention can be synthesized by subjecting, to an addition-dehydration reaction, an alkenyl-substituted nadic anhydride of the following General Formula (3) and a dimer diamine obtained by substituting two carboxyl groups of a dimer acid with a primary amino group or a primary aminomethyl group.

### (Alkenyl-substituted nadic anhydride)

An alkenyl-substituted nadic anhydride used to synthesize a bisalkenyl-substituted nadimide according to the present invention is a compound having a structure of the following General Formula (3).

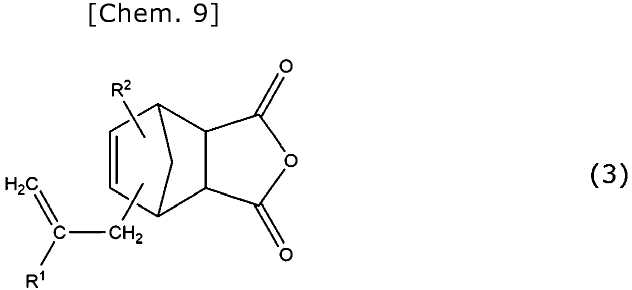

In Formula (3) above, R¹ and R² are each a hydrogen atom or a C₁-C₆ alkyl group selected independently, preferably each a hydrogen atom or a C₁-C₃ alkyl group selected independently, more preferably each a hydrogen atom or a methyl group selected independently, still more preferably each a hydrogen atom.

As the alkenyl-substituted nadic anhydride of Formula (3) above, any of various known alkenyl-substituted nadic anhydrides can be used without particular limitation. Typical examples comprise allylbicyclo[2.2.1]hepto-5-ene-2,3-dicarboxylic anhydride, methacrylbicyclo[2.2.1]hepto-5-ene-2,3-dicarboxylic anhydride, allylmethylbicyclo[2.2.1]hepto-5-ene-2,3-dicarboxylic anhydride, and methacrylmethylbicyclo[2.2.1]hepto-5-ene-2,3-dicarboxylic anhydride. These compounds may each be a mixture of geometric isomers of the compound.

In this regard, an alkenyl-substituted nadic anhydride can be produced easily, using a known method. For example, allylbicyclo[2.2.1]hepto-5-ene-2,3-dicarboxylic anhydride can be produced by dropping, into a methylethylketone solution of a maleic anhydride, allylcyclopentadiene obtained from allyl chloride and cyclopentadiene, and allowing the resultant mixture to react.

### (Dimer diamine)

A dimer diamine is a compound from which the divalent hydrocarbon group A of a bisalkenyl-substituted nadimide according to the present invention is derived.

A dimer diamine is a compound derived from a dimer acid, which is a dimer of an unsaturated fatty acid such as oleic acid, for example, as described in JPH09-12712A. Dimer acids exist, not having a single structure but having a plurality of structures or isomers. Dimer diamines, which are derivatives of dimer acids, also exist, not having a single structure but having a plurality of structures or isomers. Specific examples of the structure comprise, but are not limited to, the following.

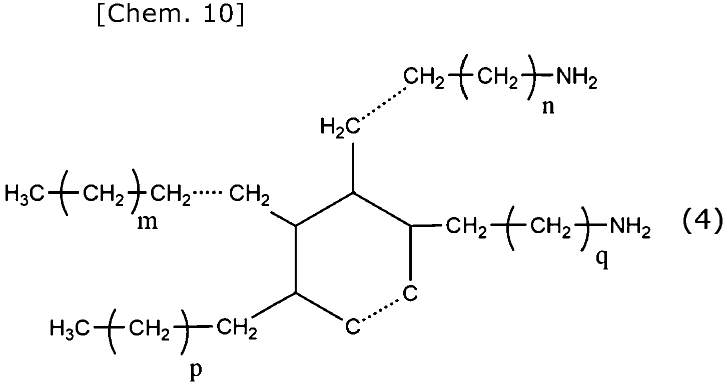

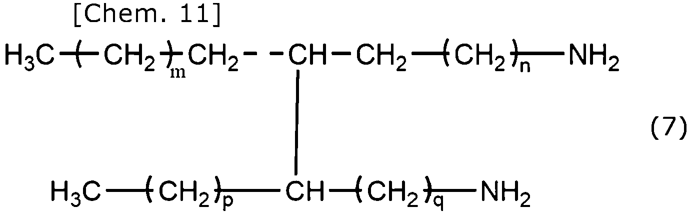

In Formulas (4) and (7), m, n, p, and q are each an integer of 1 or greater selected so as to satisfy m + n = 5 to 16 and p + q = 8 to 19; and the bonds shown by the dashed lines each mean a carbon-carbon single bond or a carbon-carbon double bond, with the proviso that, in a case where the bond shown by each dashed line is a carbon-carbon double bond, Formulas (4) and (7) are each a structure in which the number of hydrogen atoms bound to each of the carbon atoms constituting the carbon-carbon double bond is determined by subtracting 1 from each number shown in Formulas (4) and (7).

In the present embodiment, a known dimer diamine can be used without particular limitation. A dimer diamine in the present invention preferably comprises, for example, at least one of a compound of Formula (4) or a compound of Formula (7), more preferably comprises a compound of Formula (4).

Such a known dimer diamine may be a commercially available product. Examples of the commercially available product comprise: "VERSAMINE 551" and "VERSAMINE 552" (which are tradenames, manufactured by BASF Japan Ltd.); and "PRIAMINE 1073", "PRIAMINE 1074", and "PRIAMINE 1075" (which are tradenames, manufactured by Croda Japan Co., Ltd.).

In the present invention, the addition-dehydration reaction between an alkenyl-substituted nadic anhydride and a dimer diamine is performed basically through a stoichiometric reaction between both of the components. For example, the reaction is performed by heating a dimer diamine compound in an amount of 1.0 mol and an alkenyl-substituted nadic anhydride in an amount ranging from 1.5 to 3.0 mol, more preferably from 1.7 to 2.5 mol, in the presence of or in the absence of a solvent at a temperature of 20 to 220°C, preferably 100 to 160°C, for 0.5 to 20 hours, preferably 2 to 10 hours while water generated by the reaction is removed out of the system.

The reaction is performed preferably in the presence of a solvent. Examples of the solvent to be used comprise aliphatic hydrocarbons, alicyclic hydrocarbons, aromatic hydrocarbons, halogenides of these hydrocarbons, oxygen-containing, nitrogen-containing, or sulfur-containing compounds of these hydrocarbons, and solvent mixtures thereof. Specific examples comprise, but are not limited to: aliphatic hydrocarbons such as hexane and heptane; alicyclic hydrocarbons such as cyclohexane and methylcyclohexane; aromatic hydrocarbons such as benzene, toluene, xylene, methylnaphthalene, and tetralin; halogenated hydrocarbons such as chloroform, trichlene, tetrachloroethylene, chlorobenzene, and o-dichlorobenzene; oxygen-containing compounds such as dioxane, tetrahydrofuran, anisole, acetone, methylethylketone, methylisobutylketone, and acetophenone; nitrogen-containing compounds such as N-methyl-2-pyrrolidone and N,N-dimethyl formamide; and sulfur-containing compounds such as dimethyl sulfoxide.

### [Method of curing bisalkenyl-substituted nadimide]

A bisalkenyl-substituted nadimide according to the present invention, when heated, undergoes a crosslinking reaction. On the other hand, it is presumed that the retro-Diels-Alder reaction of alkenyl norbornene occurs together, and a component separated is generated in gas form, becoming the cause of voids in a cured product in some cases. A bisalkenyl-substituted nadimide according to the present invention has a low viscosity at approximately 200°C or less, and thus, is preferably heated preliminarily in this temperature range, and defoamed beforehand so as not to cause the cured product to generate voids. To facilitate the defoaming, a reduced-pressure oven is also preferably used for the heating. The heating time is taken preferably until almost no generation of voids is observed. Then, further raising the temperature causes gelation to progress rapidly, causing curing to progress. For the final curing, further heating is performed at 200°C or more and 400°C or less, preferably 200°C or more and 300°C or less. To inhibit ununiform curing, volume shrinkage, and generation of voids in the cured product, the heating is also preferably performed stepwise. The curing is performed at the final curing temperature for 1 hour or more, preferably 5 hours or more, more preferably 10 hours or more, still more preferably 20 hours or more, thereby making it possible to obtain a cured product having excellent heat resistance, mechanical characteristics, and electric characteristics.
In addition, a curing catalyst may be added to facilitate the crosslinking reaction during the curing.

### (Curing catalyst)

For the curing of a bisalkenyl-substituted nadimide according to the present invention, a curing catalyst, for example, an organic peroxide, onium salt, cationic catalyst, organic group-containing metal compound, and amine compound may be added to facilitate the crosslinking reaction.

Examples of the organic peroxide comprise, but are not limited to, di-t-butyl peroxide, di-t-amyl peroxide, dicumyl peroxide, t-butylcumyl peroxide, diacetyl peroxide, dipropionyl peroxide, di-i-butyryl peroxide, benzoyl peroxide, peroxysuccinic acid, t-butyl hydroperoxide, cyclohexyl hydroperoxide, cumene hydroperoxide, t-butyl peroxybenzoate, t-butyl peroxypivalate, 1,1-di-t-butyl peroxycyclohexane, di-t-butyl peroxyisophthalate, t-butyl peroxymaleate, t-butyl peroxyisopropylcarbonate, and 2,2-di-t-butyl peroxybutane.

Examples of the onium salt comprise, but are not limited to: ammonium compounds such as benzyltriethylammonium chloride, benzyltriethylammonium bromide, phenyltrimethylammonium bromide, tetra-n-butylammonium chloride, tetra-n-butylammonium perchlorate, tetraethylammonium tetrafluoroborate, m-trifluoromethylphenyltrimethylammonium bromide, and tetra-n-butylammonium trifluoromethanesulfonate; phosphonium compounds such as methyltriphenylphosphonium iodide, methyltriphenylphosphonium bromide, benzyltriphenylphosphonium chloride, tetraphenylphosphonium bromide, and 3-bromopropyltriphenylphosphonium bromide; arsonium compounds such as benzyltriphenylarsonium chloride, tetraphenylarsonium bromide, and tetra-n-butylarsonium chloride; stibonium compounds such as benzyltriphenylstibonium chloride and tetraphenylstibonium bromide; oxonium compounds such as triphenyloxonium chloride and triphenyloxonium bromide; sulfonium compounds such as triphenylsulfonium tetrafluoroborate, triphenylsulfonium hexafluoroarsenate, tri(p-methoxyphenyl)sulfonium hexafluorophosphate, tri(p-tolyl)sulfonium tetrafluoroborate, dimethylphenacylsulfonium hexafluorophosphate, and dimethylphenacylsulfonium hexafluoroborate; selenonium compounds such as triphenylselenonium tetrafluoroborate, triphenylselenonium hexafluoroarsenate, triphenylselenonium hexafluoroantimonate, and p-(t-butylphenyl)diphenylselenonium hexafluoroarsenate; stannonium compounds such as triphenylstannonium chloride, triphenylstannonium bromide, tri-n-butylstannonium bromide, and benzyldiphenylstannonium chloride; and iodonium compounds such as diphenyliodonium chloride, diphenyliodonium bromide, diphenyliodonium perchlorate, diphenyliodonium tetrafluoroborate, diphenyliodonium hexafluoroarsenate, diphenyliodonium hexafluorophosphate, diphenyliodonium hexafluoroantimonate, (p-methoxyphenyl)phenyliodonium tetrafluoroborate, di(2-nitrophenyl)iodonium hexafluoroarsenate, di(p-tolyl)iodonium hexafluorophosphate, and di(p-chlorophenyl)iodonium hexafluoroarsenate.

Examples of the cationic catalyst comprise, but are not limited to; acids, Bronsted acids which release acids, and esters and amine complexes thereof, such as sulfuric acid, dimethylsulfuric acid, diethylsulfuric acid, pyridinesulfuric acid, phosphoric acid, phosphorous acid, phenylphosphonic acid, phenylphosphinic acid, triethyl phosphate, dimethyl phosphate, phenyl phosphite, methanesulfonic acid, trifluoromethanesulfonic acid, m-xylene-4-sulfonic acid, p-xylene-2-sulfonic acid, benzenesulfonic acid, p-toluenesulfonic acid, a p-toluenesulfonic acid-triphenyl amine complex, a p-toluenesulfonic acid-pyridine complex, an m-nitrobenzenesulfonic acid-pyridine complex, naphthalenesulfonic acid, methyl benzenesulfonate, and ethyl p-toluenesulfonate; halogenides exhibitive of Lewis acidity or complexes with bases thereof, such as boron trichloride, boron trifluoride, a boron trifluoride-ether complex, iron trichloride, tin tetrachloride, titanium tetrachloride, aluminium chloride, an aluminium chloride-pyridine complex, aluminium bromide, zinc chloride, and antimony pentachloride.

Examples of the orgainic group-containing metal compound comprise, but are not limited to: acetyl acetone salts of, carboxylic acid salts of, metallocenes of, alcoholates of, chelate compounds of, and organic metal compounds of a transition element such as magnesium, zinc, titanium, vanadium, chromium, manganese, iron, cobalt, nickel, copper, zirconium, molybdenum, ruthenium, rhodium, lanthanum, cerium, hafnium, tantalum, or tungsten, preferably magnesium, zinc, vanadium, manganese, iron, or cerium; still more preferably acetyl acetone salts of or carboxylic acid salts of magnesium, zinc, vanadium, manganese, iron, or cerium.

Examples of the amine compound comprise: a heterocyclic amine, aliphatic tertiary amine, aromatic tertiary amine, Lewis acid complex thereof, and dihydrazide compound. Examples of the heterocyclic amine comprise: imidazole, pyridine, dimethyl aminopyridine, piperidine, piperazine, and a substitution product thereof with an alkyl group, alkenyl group, phenyl group, or benzyl group, or an N-acyl. Examples of the imidazole substitute comprise 2-methylimidazole, 2-ethyl-4-methylimidazole, 2-vinylimidazole, 2-phenylimidazole, 2-phenyl-4-methylimidazole, 1-(3-aminopropyl)imidazole, 1-(2,4,6-trimethylbenzoyl)-2-phenylimidazole, 1-(2,6-dichloro benzoyl)-2-methylimidazole, 1-(2,6-dichlorobenzoyl)-2-phenylimidazole, 1-(2,6-dichlorobenzoyl)-2-ethyl-4-methylimidazole, 1-(2,6-dichlorobenzoyl)-4-phenylimidazole, 1-(2-chloro-6-nitrobenzoyl)-2-ethylimidazole, 1-pentachlorobenzoyl-2-methylimidazole, 1-pentachlorobenzoyl-2-phenylimidazole, 1-propenyl-2-phenylimidazole, and 1-propenyl-4-phenylimidazole. In addition, examples of the aliphatic tertiary amine comprise triethylamine, diethylaminopropylamine, diethylaminoethylamine, dimethylaminobenzylamine, 1,6-bis(dimethylamino)hexane, and triethanolamine. Examples of the aromatic tertiary amine comprise, but are not limited to, N,N-dimethylaniline and besides, N,N-dimethylbenzylamine, N,N-diethylbenzylamine, N,N-dimethylaminobenzaldehyde, and tris(dimethylaminomethyl)phenol. Examples of the Lewis acid of the Lewis acid complex of the tertiary amine comprise, but are not limited to, BF₃, BCl₃, AlCl₃, FeCl₃, TiCl₄, and SnCl₄, in particular BF₃ and BCl₃. In addition, as a typical dihydrazide compound, a compound of the following General Formula (8) is used.

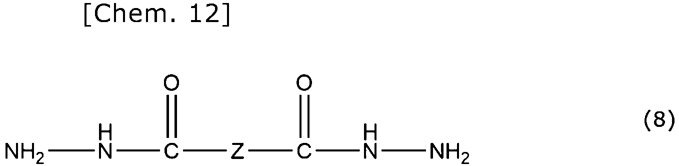

In Formula (8), Z is a single bond or a divalent organic group, in particular a group of an aliphatic, cycloaliphatic, or aromatic dicarboxylic acid.

Specific examples of Formula (8) above comprise, but are not limited to, dihydrazide oxalate, dihydrazide malonate, dihydrazide succinate, dihydrazide glutarate, dihydrazide adipate, dihydrazide pimelate, dihydrazide suberate, dihydrazide sebacate, dihydrazide cyclohexanedicarboxylate, dihydrazide terephthalate, dihydrazide isophthalate, and dihydrazide 2,6- or 2,7-naphthalenedicarboxylate, and in addition, comprise mixtures thereof.

In addition, the amine compounds which can be used are not only the above-described compounds but also dicyandiamide and a compound of the following Formula:

(CH₃)₂N-(CH₂)₃-NH(CH₂)₃-NH₂,

(CH₃)₂N-(CH₂)₃-NH(CH₂)₃-CN,

or

(CH₃)₂N-(CH₂)₃-N(CH₂CH₂CN)₂.

The amount of any of these catalysts is not particularly limited, and may be suitably selected in accordance with the form of usage.

### [Curable material]

A curable material according to the present invention comprises a bisalkenyl-substituted nadimide according to the present invention, and may further comprise another component such as a curing catalyst. A bisalkenyl-substituted nadimide according to the present invention may be used singly as a curable material without being combined with another resin.

### [Curable resin composition]

As described above, a bisalkenyl-substituted nadimide according to the present invention is used singly as a curable material for various uses, and besides, may be combined with any of various other compounds, resins, and the like to be formed into a curable resin composition. Among others, a curable resin composition comprising the component (A) and the component (B) has not only excellent dielectric characteristics, flexibility, and low water-absorbing properties but also excellent heat resistance and toughness, and can yield a particularly useful cured product in a laminating material, molding material, and composite material, wherein the component (A) is a bisalkenyl-substituted nadimide according to the present invention, and the component (B) is at least one compound of a maleimide compound, an alkenyl-substituted nadimide (excluding the bisalkenyl-substituted nadimide according to the present invention), an epoxy resin, a phenol resin, a vinylbenzyl compound, a vinyl compound (excluding a vinylbenzyl compound), a cyclic olefin, a conjugated diene resin having a functional group, or an unsaturated polyester resin.

Examples of the maleimide compound as the component (B) to be used for the curable resin composition comprise an aliphatic maleimide, aromatic maleimide, aliphatic citraconimide, aromatic citraconimide, and bismaleimide. Out of these, examples of the aliphatic maleimide comprise maleimide, N-methylmaleimide, N-ethylmaleimide, N-propylmaleimide, N-laurylmaleimide, N-(2-hydroxyethyl)maleimide, and N-cyclohexylmaleimide. Examples of the aromatic maleimide comprise N-phenylmaleimide, N-(2-methylphenyl)maleimide, N-(3-ethylphenyl)maleimide, N-(4-methylphenyl)maleimide, N-(2,6-diethylphenyl)maleimide, N-(2,4,6-trimethylphenyl)maleimide, N-(2-chlorophenyl)maleimide, N-(4-chlorophenyl)maleimide, N-(4-hydroxyphenyl)maleimide, N-(4-hydroxymethylphenyl)maleimide, N-(4-carboxyphenyl)maleimide, N-(4-ethoxycarbonylphenyl)maleimide, N-(4-benzylphenyl)maleimide, and N-(4-phenoxyphenyl)maleimide. Examples of the aliphatic citraconimide comprise citracon imide, N-methylcitraconimide, N-butylcitraconimide, and N-cyclohexylcitraconimide. Examples of the aromatic citracon imide comprise N-phenylcitraconimide, N-(3-ethylphenyl)citraconimide, N-(2,6-dimethylphenyl)citraconimide, N-(4-chlorophenyl)citraconimide, N-(4-hydroxyphenyl)citraconimide, and N-(4-benzylphenyl)citraconimide.

In particular, a bismaleimide compound of the following General Formula (5) is added to a bisalkenyl-substituted nadimide according to the present invention, thereby enabling the curable resin composition to further achieve low-temperature curability. 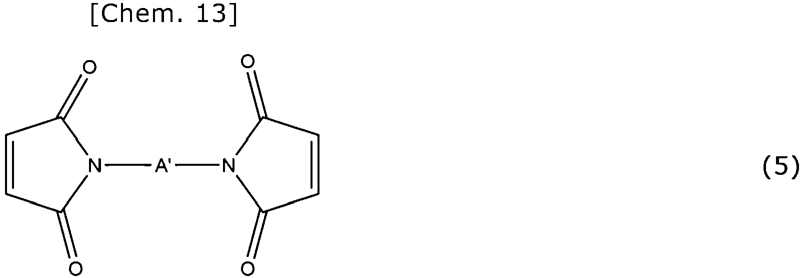 wherein, in Formula (5), A' is a divalent hydrocarbon group.

Examples of the bismaleimide comprise bis(4-maleimidephenyl)methane, N,N'-phenylenebismaleimide, bis(4-maleimidephenyl)ether, bis(4-maleimidephenyl)sulfone, bis(4-maleimidecyclohexyl)methane, N,N'-tolylenebismaleimide, N,N'-dimethyl phenylenebismaleimide, N,N'-xylylenebismaleimide, bis(4-maleimidephenyl)cyclohexane, N,N'-dichlorobiphenylenebismaleimide, bis[4-(methylmaleimide)phenyl] methane, bis[4-(methylmaleimide)phenyl]ether, bis[4-(methylmaleimide)phenyl] sulfone, N,N'-ethylenebismaleimide, N,N'-hexamethylenebismaleimide, and N,N'-hexamethylenebismethylmaleimide. In addition, A' may be, but is not limited to, a structure derived from a dimer acid skeleton. These bismaleimides may be used singly or in mixture of a plurality of kinds thereof, and furthermore, may be used in the form of an oligomer.

In addition, the alkenyl-substituted nadimide to be used as the component (B) may be other than the novel alkenyl-substituted nadimide according to the present invention of General Formula (1), i.e., may be a reaction product between an amine other than a dimer diamine and an alkenyl-substituted nadic anhydride.

Specific examples of the bisalkenyl-substituted nadimide synthesized using a diamine other than a dimer diamine comprise, but are not limited to, N-allyl-allylbicyclo[2.2.1]hepto-5-ene-2,3-dicarboxyimide, N-(3'-hydroxyphenyl)-allylbicyclo[2.2.1]hepto-5-ene-2,3-dicarboxyimide, N-(4'-carboxyphenyl)-allylbicyclo[2.2.1]hepto-5-ene-2,3-dicarboxyimide, N,N'-ethylene-bis(allylbicyclo[2.2.1]hepto-5-ene-2,3-dicarboxyimide), N,N'-hexamethylene-bis(allylbicyclo[2.2.1]hepto-5-ene-2,3-dicarboxyimide), N,N'-m-phenylene-bis(allylbicyclo[2.2.1]hepto-5-ene-2,3-dicarboxyimide), N,N'-m-xylylene-bis(allylbicyclo[2.2.1]hepto-5-ene-2,3-dicarboxyimide), N,N'-p-xylylene-bis(allylbicyclo[2.2.1]hepto-5-ene-2,3-dicarboxyimide), bis[4-(allylbicyclo[2.2.1]hepto-5-ene-2,3-dicarboxyimide)phenyl]methane, bis[4-(allylbicyclo[2.2.1]hepto-5-ene-2,3-dicarboxyimide)phenyl]ether, bis[4-(allylbicyclo[2.2.1]hepto-5-ene-2,3-dicarboxyimide)phenyl]sulfone, 1,6-bis(allylbicyclo[2.2.1]hepto-5-ene-2,3-dicarboxyimide)-3-hydroxy-hexane, N,N'-2,5-dimethylene-bicyclo[2.2.1]heptane-bis(allylbicyclo[2.2.1]hepto-5-ene-2,3-dicarboxyimide, and N,N'-3,8-dimethylene-tricyclo[5.2.1.0]decane-bis(allylbicyclo[2.2.1]hepto-5-ene-2,3-dicarboxyimide. The above-described examples are allyl-substituted nadimides. Obviously, a methallyl-substituted, allylmethyl-substitutd, or methallylmethyl-substituted nadimide may be used. The alkenyl-substituted nadimide having a such structure may be used singly, or may be used in mixture thereof or in the form of an oligomer.

In addition, the epoxy resin usable as the component (B) is any of various known epoxy resins. Examples thereof comprise, but are not limited to: a bisphenol epoxy resin obtained by reaction between bisphenol and epichlorohydrin; a novolac epoxy resin obtained by reaction between a phenol novolac resin or a cresol novolac resin and epichlorohydrin; a polyhydroxybenzene epoxy resin obtained by reaction between a multivalent phenol such as resorcinol or hydroquinone and epichlorohydrin; a nitrogen-containing epoxy resin obtained by reaction between an aromatic amine such as toluidine or aniline and epichlorohydrin; an alicyclic epoxy resin obtained, using cyclohexene, vinyl cyclohexene, cyclopentadiene, dicyclopentadiene, or the like as a starting raw material; an epoxy resin having a fluorene skeleton; a di- or poly-glycidyl ether such as butanediol, hexanediol, glycerin, trimethylolpropane, or pentaerythritol; a silicon-containing epoxy resin obtained by allowing silanol to react with an epoxy resin; and an epoxy resin having a nitrogen-containing hetero ring, and obtained from isocyanuric acid or cyanic acid amide. In addition, these epoxy resins may be used in mixture of two or more kinds thereof.

In addition, the phenol resin usable as the component (B) is any of various known phenol resins. Examples thereof comprise; a common phenol resin produced from a phenolic compound and a formaldehyde-supplying substance; besides, an aromatic hydrocarbon resin such as a xylene-formaldehyde resin; a reaction product between a xylene resin and phenol; a phenol alkyl resin synthesized by reaction between phenol and dimethoxyxylene; a phenol-dicyclopentadiene resin obtained by reaction between phenol and dicyclopentadiene; a resin obtained by polymerizing paravinyl phenol, isopropyl phenol, or the like. In addition, the resins may be used in modified form or in mixture of several kinds thereof. Furthermore, as the phenol resin, a novolac type thereof or a resole type thereof can be used. The novolac type is preferable from the viewpoint of compatibility with the component (A) and the other component (B). The number-average molecular weight of the phenol resin is 200 to 2000, preferably 300 to 900. In this regard, examples of the phenol compound as a raw material for the common phenol resin comprise phenol, cresol, xylenol, ethylphenol, p-t-butylphenol, p-octylphenol, p-nonylphenol, p-cumylphenol, p-phenylphenol, hydroquinone, resorcin, catechol, bisphenol A, bisphenol F, methylcatechol, butylcatechol, cresorcin, methylhydroquinone, pyrogallol, dihydroxynaphthalene, trihydroxynaphthalene, and mixtures thereof.

In addition, the vinylbenzyl compound usable as the component (B) is any of various known vinylbenzyl compounds of General Formula (9): 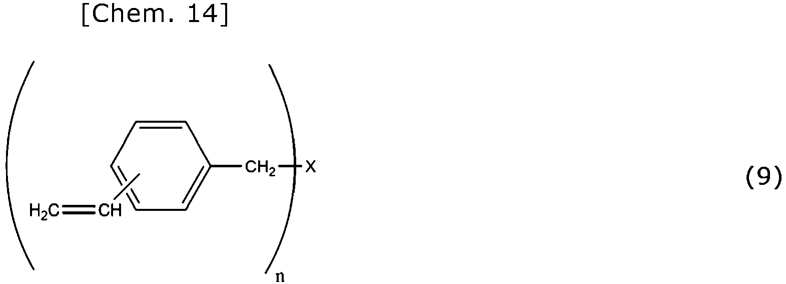 in Formula (9), n is an integer of 1 or 2; in a case where n = 1, X is a residue resultant from removing one hydrogen atom from the amino group of a C₆-C₁₈ monovalent aromatic primary or secondary amine (for example, aniline, naphthyl amine, N-ethylaniline, diphenylamine, N-methylnaphthylamine, N-phenylnaphthylamine, dinaphthylamine, carbazole, and the like), a residue resultant from removing one hydrogen atom from the amino group of a C₂-C₁₂ monovalent aliphatic primary or secondary amine (for example, ethylamine, propylamine, hexylamine, decylamine, diethylamine, dibutylamine, N-propylhexylamine, cyclohexylamine, cyclooctylamine, N-ethylcyclohexylamine, and the like), or a residue resultant from removing the hydrogen atom from the hydroxyl group of a C₆-C₁₈ monovalent phenol (for example, phenol, ethylphenol, naphthol, methylnaphthol, p-phenylphenol, and the like); and in a case where n = 2, X is oxygen, a residue resultant from removing two hydrogen atoms from the amino group of a C₆-C₁₈ monovalent aromatic primary amine (for example, aniline, naphthylamine, and the like) or a C₂-C₁₂ monovalent aliphatic primary amine (for example, ethylamine, propylamine, hexylamine, decylamine, cyclohexylamine, cyclooctylamine, and the like), a residue resultant from removing two hydrogen atoms from the amino group of a C₆-C₁₈ multivalent aromatic primary or secondary amine (for example, phenylenediamine, diaminonaphthalene, benzidine, N-methylphenylenediamine, N-phenyldiaminonaphthalene, N-methyl benzidine, and the like) (i.e., a residue resultant from removing two hydrogen atoms from one amino group or a residue resultant from removing one hydrogen atom from each of two amino groups), a residue resultant from removing two hydrogen atoms from the amino group of a C₂-C₁₂ multivalent aliphatic primary or secondary amine (for example, ethylenediamine, hexamethylenediamine, diaminocyclohexane, N-methylethylenediamine, and the like) (i.e. a residue resultant from removing two hydrogen atoms from one amino group or a residue resultant from removing one hydrogen atom from each of two amino groups), or a residue resultant from removing a hydrogen atom from each of two hydroxyl groups of a C₆-C₁₈ multivalent phenol (for example, hydroquinone, resorcin, catechol, biphenol, bisphenol A, bisphenol F, dihydroxybenzophenone, methylcatechol, butylcatechol, cresorcin, methylhydroquinone, pyrogallol, dihydroxynaphthalene, trihydroxynaphthalene, and the like). Examples of the vinylbenzyl compound comprise, but are not limited to, N-vinylbenzylaniline, N,N'-bis(vinylbenzyl)aniline, bis(vinylbenzyl)ether, N,N'-bis(vinylbenzyl)piperazine, and 2,2-bis[4'-(vinylbenzyloxy)phenyl]propane.

In addition, the vinyl compound (excluding a vinylbenzyl compound) to be used as the component (B) is any of various known compounds having one or more vinyl groups. Examples thereof comprise an unsaturated aromatic hydrocarbon, an unsaturated carboxylic acid, an ester of an unsaturated carboxylic acid and a saturated alcohol, an ester of a saturated and/or unsaturated carboxylic acid and an unsaturated alcohol, an ester of an isocyanuric acid and an unsaturated alcohol, an ether compound, a nitrogen-containing compound, a sulfur-containing compound, a zinc-containing compound, and a silicon-containing compound. Specific examples of the vinyl compound comprise, but are not limited to: unsaturated aromatic hydrocarbons such as styrene, α-methylstyrene, ethylvinylbenzene, o-, m-, and p-divinylbenzenes, and divinylnaphthalene; unsaturated carboxylic acids such as acrylic acid, methacrylic acid {hereinafter, the acrylic acid and methacrylic acid compounds are expressed in common by "(meth)"}, maleic acid, maleic anhydride, fumaric acid, itaconic acid, and itaconic anhydride; esters of an unsaturated carboxylic acid and a saturated alcohol, such as methyl (meth)acrylate, ethyl (meth)acrylate, 2-hydroxyethyl (meth)acrylate, 2-ethoxyethyl (meth)acrylate, tetrahydrofurfuryl (meth)acrylate, 2-phenoxyethyl (meth)acrylate, isodecyl (meth)acrylate, isobornyl (meth)acrylate, cyclohexyl (meth)acrylate, lauryl (meth)acrylate, stearyl (meth)acrylate, glycidyl (meth)acrylate, ethylene glycol di(meth)acrylate, diethylene glycol di(meth)acrylate, triethylene glycol di(meth)acrylate, tetraethylene glycol di(meth)acrylate, tripropylene glycol di(meth)acrylate, neopentyl glycol di(meth)acrylate, propoxylated neopentyl glycol di(meth)acrylate, 1,3-butylene glycol di(meth)acrylate, 1,4-butanediol di(meth)acrylate, 1,6-hexane diol di(meth)acrylate, ethoxylated bisphenol A di(meth)acrylate, tetrabromobisphenol A diethoxylated di(meth)acrylate, and tricyclodecanedimethylol di(meth)acrylate; esters of a saturated and/or unsaturated carboxylic acid and an unsaturated alcohol, such as vinyl acetate, vinyl benzoate, vinyl phthalate, divinyl adipate, allyl (meth)acrylate, di-2-propenyl 1,2-benzenedicarboxylate, di-2-propenyl 1,3-benzenedicarboxylate, diallyl phthalate, diallyl isophthalate, diallyl maleate, and diethylene glycol bisallyl carbonate; esters of an isocyanuric acid and an unsaturated alcohol, such as triallyl isocyanurate and trimethallyl isocyanurate; ether compounds such as 1,1'-oxybisethane and 3,3'-oxybis-1-propene; nitrogen-containing compounds such as acrylonitrile, N-2-propenyl-2-propene-1-amine, and 2,2-dichloro-N,N-di(2-propenyl)acetamide; sulfur-containing compounds such as 1,1'-thobisethane, 1,1'-sulfonylbisethane, and di-1-propenyl disulfide; zinc-containing compounds such as zinc diacrylate and zinc dimethacrylate; silicon-containing compounds such as 1,3-divinyl-1,1,3,3-tetramethyldisiloxane; and besides, 3,9-divinyl-2,4,8,10-tetraoxaspiro[5.5]undecane and dialkenylbisphenol A. Among these, o-, m-, and p-divinyl benzenes, di-2-propenyl 1,3-benzene dicarboxylate, di-2-propenyl 1,2-benzene dicarboxylate, diallyl phthalate, and triallyl isocyanurate are preferably used from the viewpoints of the reactivity of the resultant composition, the workability during preparation of the composition, and the like.

In addition, the cyclic olefin usable as the component (B) is any of various known cyclic olefins. Examples thereof comprise, but are not limited to: cyclopentene, cyclohexene, 4-vinylcyclohexene, cycloheptene, cyclooctene, cyclododecene, cyclopentadiene, dicyclopentadiene, 1,3-cyclohexadiene, 1,3-cycloheptadiene, 1,5-cyclooctadiene, and indene.

In addition, the conjugated diene resin having a functional group, and usable as the component (B) is any of various known conjugated diene resins having a functional group, and is, for example, commonly a C₄-C₁₂ conjugated diene. Examples thereof comprise: a polymer obtained by introducing a hydroxyl group, epoxy group, carboxyl group, or cyano group into a polymer such as 1,3-butadiene, isoprene, 2,3-dimethyl-1,3-butadiene, 1,2-pentadiene, 1,3-pentadiene, 1,4-pentadiene, 1,3-hexadiene, 4,5-diethyl-1,3-octadiene, or 3-butyl-1,3-octadiene; and a copolymer of any of these conjugated diene monomers and a polar vinyl monomer such as acrylonitrile, acrylic acid, or acrylic acid ester.

In addition, the unsaturated polyester resin usable as the component (B) is any of various known unsaturated polyester resins. Common examples thereof comprise a product obtained by esterification reaction between a C₂-C₁₂ low-molecular-weight diol and a C₃-C₁₂ dicarboxylic acid containing at least 50 mol% ethylenic unsaturation or an anhydride thereof. Examples of the diol comprise 1,2-propylene glycol, ethylene glycol, 1,3-propylene glycol, diethylene glycol, di-1,2-propylene glycol, 1,4-butanediol, and neopentyl glycol. The diols are used singly or in mixture. As the acid, for example, maleic acid, fumaric acid, phthalic acid, terephthalic acid, isophthalic acid, adipic acid, glutaric acid, and/or an anhydride thereof is/are used, often used in mixture, but not limited thereto.

The component (A) and the component (B) in the above-described curable resin composition are blended at a blending ratio as follows: usually 98 to 5 parts by weight of the component (A) and 2 to 95 parts by weight of the component (B); preferably 90 to 10 parts by weight of the component (A) and 10 to 90 parts by weight of the component (B); more preferably 60 to 10 parts by weight of the component (A) and 40 to 90 parts by weight of the component (B); still more preferably 55 to 15 parts by weight of the component (A) and 45 to 85 parts by weight of the component (B).

In addition, the curable resin composition comprising the component (A) and the component (B) is cured by heating alone, but to increase the curing rate, a component (C) as a curing catalyst can be used in combination, if desired. As this curing catalyst, the above-described curing catalyst used during the curing of a bisalkenyl-substituted nadimide according to the present invention can be used. The amount of the catalyst is not particularly specified, and may be suitably selected in accordance with the form of usage.

In addition, the composition is useful as a matrix of a composite material. During curing, 10 to 500 parts by weight of any of various inorganic fillers, for example, glass fiber, carbon fiber, metallic fiber, ceramic fiber, phosphoric acid calcium, calcium carbonate, magnesium carbonate, aluminium hydroxide, magnesium hydroxide, antimony oxide, gypsum, silica, alumina, clay, talc, quartz powder, and carbon black, may be mixed with 100 parts by weight of the bisalkenyl-substituted nadimide, if desired.

A cured product of the curable resin composition comprising the component (A) and the component (B) has the same heat resistance and mechanical properties as a curable resin composition produced using a conventional alkenyl-substituted nadimide, but yields a cured product improved in low dielectric constant, flexibility, and low water-absorbing properties.

Furthermore, a bisalkenyl-substituted nadimide according to the present invention may be a curable resin composition comprising the component (A) and a component (D) composed of at least one of a silicone resin, modified silicone resin, polysulfone resin, polyphenylene sulfide resin, or fluorine resin. The curable resin composition can be used, for example, as stain-resistant paint for forming a stain-resistant cured coating on a base material.

As the component (D), any of various known silicone resins, modified silicone resins, polysulfone resins, polyphenylene sulfide resins, and fluorine resins can be widely used. Specific examples thereof comprise: silicone resins such as a methylpolysiloxane resin, methylphenylpolysiloxane resin, and methylhydrogen polysiloxane resin; modified silicone resins such as an epoxy-modified silicone resin, polyester-modified silicone resin, acryl-modified silicone resin, and phenol-modified silicone resin; polysulfone resins such as a polysulfone resin, polyallylsulfone resin and polyether sulfone resin; fluorine resins such as a polytetrafluoroethylene resin, tetrafluoroethylene-perfluoroalkylvinylether copolymer, tetrafluoroethylene-hexafluoropropylene copolymer, tetrafluoroethylene-hexafluoropropylene-perfluoroalkylvinylether copolymer, tetrafluoroethylene-ethylene copolymer, polychlorotrifluoroethylene resin, and polyvinylidene fluoride resin. These resins may be used singly or in mixture of a plurality of kinds thereof.

The component (A) and the component (D) in the above-described curable resin composition are blended at a blending ratio, as follows: usually 98 to 5 parts by weight of the component (A) and 2 to 95 parts by weight of the component (D); preferably 80 to 20 parts by weight of the component (A) and 20 to 80 parts by weight of the component (D).

### [Method of curing curable resin composition]

A curable resin composition according to the present invention can be cured by: melting and mixing an alkenyl-substituted nadimide according to the present invention and the above-described components; then molding the resultant mixture, using a method such as cast molding, injection molding, or compression molding; or applying the mixture to an object; if desired, removing the solvent; drying the resultant object, thereby forming the object into a film; and heating the resultant film. The curing temperature and the curing time can be suitably adjusted depending on the components to be mixed and the blending ratio, and are commonly 80°C or more and 280°C or less, preferably 80°C or more and 220°C or less, and 0.01 hours or more and 5 hours or less, preferably 0.05 hours or more and 2 hours or less. In a case where an inorganic filler or the like is mixed in, the resultant mixture may be further heat-treated at 150 to 350°C for 0.5 to 30 hours, if desired.

### (Another component)

A curable material and a curable resin composition according to the present invention may comprise another component. The curable material and the curable resin composition can comprise, as an additive, any known material in the field of electronic materials, examples of which known material comprise a solvent, surface conditioner, surfactant, ultraviolet absorber, antioxidant, light stabilizer, tackifier, colloidal silica, defoaming agent, wetting agent, anti-rust agent, plasticizer, chain transfer agent, photosensitizer, dehydrating agent, weathering agent, heat stabilizer, lubricant, brightening agent, coloring agent, flame retardant, release agent, surface-treating agent, or viscosity modifier.

### [Cured product]

A cured product according to the present invention is obtained by curing the above-described curable material or curable resin composition according to the present invention. A cured product according to the present invention can be suitably used for an electronic material such as a circuit board. In particular, the cured product, having low dielectric characteristics in the 10 GHz band, can be suitably used for an electronic material satisfactory for 5G. In addition, the cured product, having flexibility, can be suitably used for a member in film form.

### EXAMPLES

The present invention will be described below in more detail with reference to Examples, but the present invention is not limited to the following Examples. In this regard, "part(s)" and "%" as used hereinafter are all by mass, unless otherwise specified.

### (Example 1: Synthesis of bisalkenyl-substituted nadimide A)

Into a reaction container with a thermometer, a stirring device, and a distilling receiver, 228.0 g of dimer diamine (PRIAMINE 1075, manufactured by Croda Japan Co., Ltd.) and 82.0 g of methylcyclohexane were introduced, and the resultant mixture was heated at 110°C. Then, 180.0 g of allylbicyclo[2.2.1]hepto-5-ene-2,3-dicarboxylic anhydride was added dropwise, and then, the resultant mixture was allowed to react under methylcyclohexane reflux at 110°C for 2 hours. During the reaction, water generated was discharged out of the system. Then, the resultant mixture was heated up to 140°C, and distilled under reduced pressure at a degree of pressure reduction of approximately 150 torr for 1 hour and subsequently at a degree of pressure reduction of approximately 25 torr for 2 hours to obtain 383.5 g of a bisalkenyl-substituted nadimide A (the yield, 99.1%) in the form of a dark brown viscous liquid.

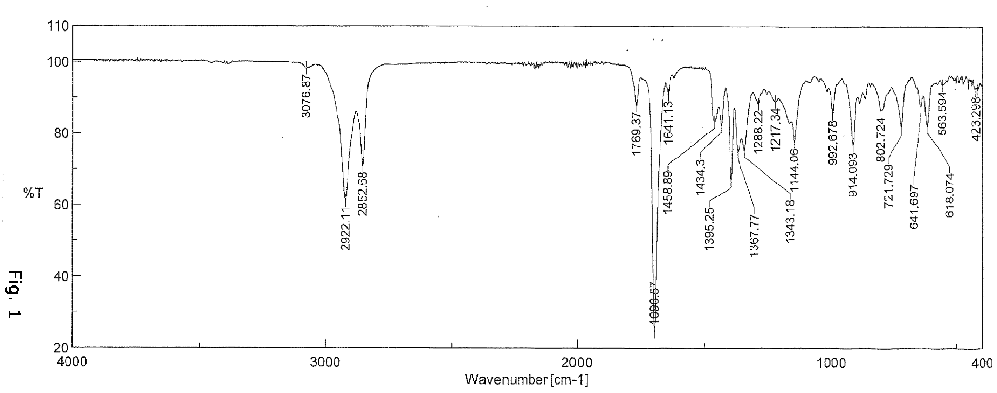

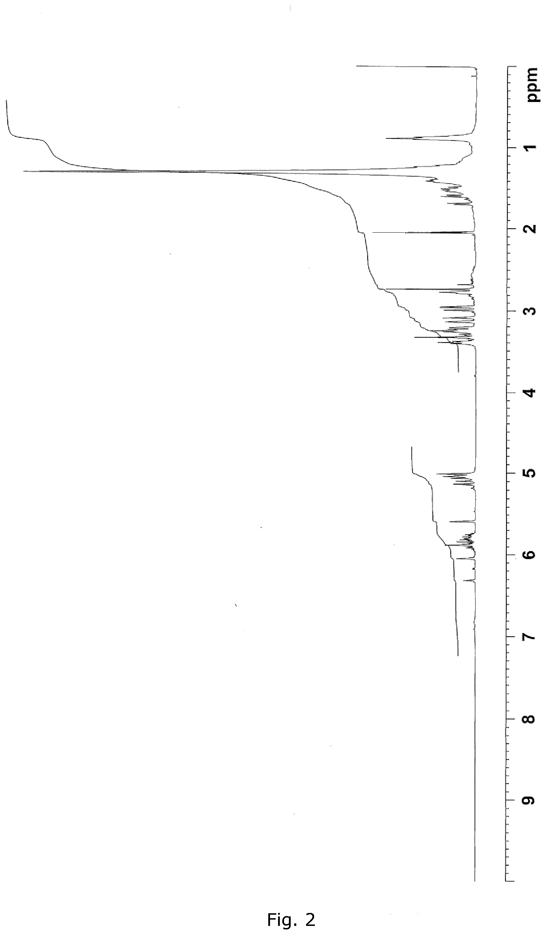

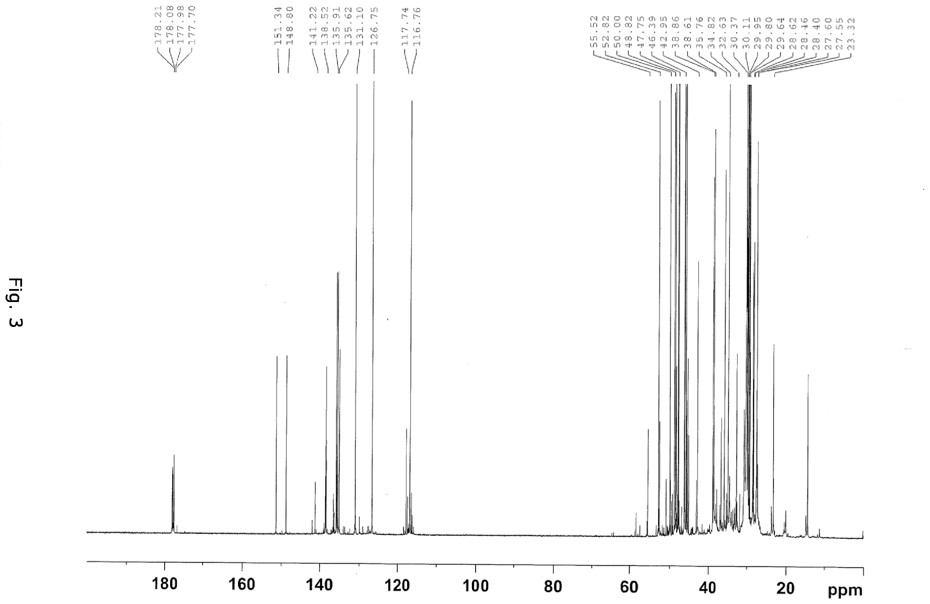

The IR spectrum, ¹H-NMR spectrum, and ¹³C-NMR (500 MHz, acetone-d6) of the bisalkenyl-substituted nadimide A synthesized in Example 1 are shown in FIG. 1 to FIG. 3 respectively.

The IR spectrum is attributed to the following:
alkene C-H stretch: 3077 cm⁻¹,
alkane C-H stretch: 2922 cm⁻¹, 2853 cm⁻¹,
imide C=O stretch: 1769 cm⁻¹, 1697 cm⁻¹,
alkene C=C stretch: 1641 cm⁻¹,
imide C-N stretch (imide II): 1396 cm⁻¹, and the like.

The acid value of the bisalkenyl-substituted nadimide A synthesized was determined through neutralization titration performed with a 0.1 M KOH/ethanol solution, using phenol phthalein as an indicator. The acid value was 8.1 (KOH mg/g).

### [Production of cured product of bisalkenyl-substituted nadimide]

### (Example 2: Production of cured product of bisalkenyl-substituted nadimide A)

Using the bisalkenyl-substituted nadimide A synthesized in Example 1, a cured product was produced as described below.
First, the bisalkenyl-substituted nadimide A was placed in a reduced-pressure oven at 200°C, and defoamed for 3 hours.

Next, the bisalkenyl-substituted nadimide A defoamed was poured into a casting mold that was fixed with clips, using two release-agent-coated glass plates and a silicone rubber 2 mm in thickness as a spacer. The resultant object was placed in the pressure-reduced oven at 200°C, and defoamed overnight.

After the overnight defoaming, the casting mold was transferred to an electric oven at 200°C under a nitrogen gas stream, heated stepwise up to 240°C, and then retained as it was for 24 hours. Then, the resultant product was cooled to approximately room temperature in the oven, and then, the resultant cured product was taken out of the mold.

### (Comparative Example 1: Production of BANI-M cured product)

The structure of BANI-M is shown by the following Formula (10).

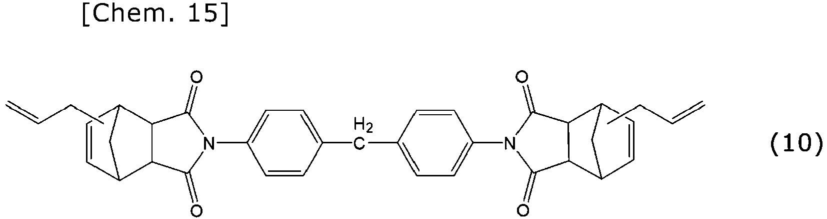

In place of the bisalkenyl-substituted nadimide A synthesized in Example 1, a bisalkenyl-substituted nadimide BANI-M (a brown solid; the melting point, 75°C) manufactured by Maruzen Petrochemical Co., Ltd. was used. The solid was defoamed in the reduced-pressure oven at 200°C for 3 hours in the same manner as in Example 2, poured into a casting mold, and defoamed in the reduced-pressure oven at 200°C overnight.

After the overnight defoaming, the casting mold was transferred to an electric oven at 200°C under a nitrogen gas stream, heated stepwise up to 250°C, and then retained as it was for 24 hours. Then, the resultant product was cooled to approximately room temperature in the oven, and then, the resultant cured product was taken out of the mold.

### (Comparative Example 2: Production of BANI-X cured product)

The structure of BANI-X is shown by the following Formula (11).

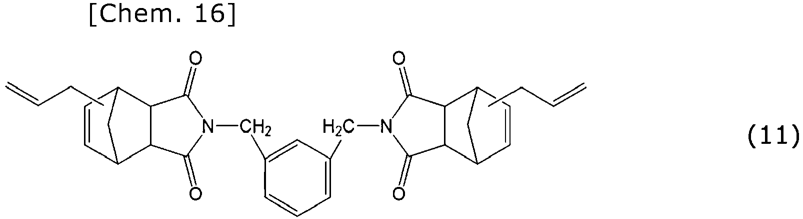

In place of the bisalkenyl-substituted nadimide A synthesized in Example 1, a bisalkenyl-substituted nadimide BANI-X (a light yellow solid; the melting point, 40°C) manufactured by Maruzen Petrochemical Co., Ltd. was used to produce a cured product in the same manner as in Comparative Example 1.

### <Evaluation of properties>

Using the cured products obtained in Example 2, Comparative Example 1, and Comparative Example 2, the following measurements were performed. The results are shown in Table 1.

### [Bending test (maximum stress and apparent elastic modulus)]

The measurements were performed under the conditions described below in accordance with JIS K7171. The bending test was performed through a measurement by crosshead displacement, and no compliance correction was made.

The apparent modulus was calculated from the stress gradient of the specified range of strain (from 0.05% to 0.25%). In each of Example 2 and Comparative Example 2, five measurements were made. In Comparative Example 1, three measurements were made. The respective averages were calculated.
Measuring device: model 5966 universal testing machine manufactured by Instron
Dimensions of test piece: 50 mm × 25 mm × 2 mm (thickness)
Testing temperature: 23°C
Testing rate: 1 mm/min
Fulcrum-to-fulcrum distance: 32 mm (the radius of the indenter, 5 mm; the radius of the support, 2 mm)

### [Measurement of ratio of water absorption]

The test piece was preliminarily dried at 50°C for 24 hours, and allowed to absorb water at an immersion temperature of 23°C for an immersion time of 24 hours. The ratio of water absorption was calculated from the masses of the test piece before and after the absorption of water. Three measurements each were made, and the average was regarded as the ratio of water absorption.

### [Measurement of dielectric constant and dielectric loss factor]

The measurements were performed under the conditions described below in accordance with IEC 62810 (the cylindrical-cavity-based perturbation method).
Measuring device: PNA Network Analyzer N5222B manufactured by Keysight Technologies Inc.
Cylindrical cavity resonator: CP531 (for 10 GHz) manufactured by Kanto Electronics Application & Development Inc.
Dimensions of test piece: 80 mm × 1.5 mm × 1.5 mm
Measuring frequency: 10 GHz
Conditioning: 23°C ± 1°C, RH 50% + 5% × 24 hours
Testing environment: 23°C ± 1°C, RH 50% ± 5%
Number of measurements: n = 2

### [TMA measurement (coefficient of linear expansion and glass transition temperature)]

The TMA measurements were performed under the below-described conditions. The coefficient of linear expansion was calculated from the results obtained in the range of from 25°C to 300°C.
Measuring device: TMA8311 manufactured by Rigaku Corporation
Dimensions of test piece: 10 mm × 5 mm × 2 mm (thickness)
Temperature range: from room temperature to 400°C
Heating rate: 5°C/minute
Atmosphere: N₂ (100 mL/minute)
Mode: Compression (the load, 49 mN)

**[Table 1]**

| Measurement and T est | Evaluation Item | Unit | Example 2 | Comparative Example 1 | Comparative Example 2 |
|---|---|---|---|---|---|
| | | | A | BANI-M | BANI-X |
| Bending test | Maximum stress | MPa | 15.0 | 144 | 163 |
| | Apparent modulus | MPa | 543 | 3380 | 3700 |
| Measurement of ratio of water absorption | Ratio of water absorption | % by mass | 0.14 | 1.56 | 1.10 |
| Measurement of dielectric constant and dielectric loss factor | Dielectric constant (10 GHz) | - | 2.24 | 2.74 | 2.43 |
| | Dielectric loss factor (10 GHz) | - | 1.84 × 10⁻³ | 1.90 × 10⁻² | 2.04 × 10⁻² |
| TMA Measurement | Coefficient of linear expansion | °C | 9.4 × 10⁻⁵ | 4.5 × 10⁻⁵ | 4.5 × 10⁻⁵ |
| | Glass transition temperature | °C | 48 to 61 | 330 | 308 to 357 |

The results in Table 1 above have verified that the bisalkenyl-substituted nadimide A in Example 2 exhibits a lower ratio of water absorption than the bisalkenyl-substituted nadimide compounds in Comparative Examples 1 and 2. In addition, the results have also verified that the bisalkenyl-substituted nadimide A in Example 2 exhibits a lower dielectric constant and dielectric loss factor in the 10 GHz band than the bisalkenyl-substituted nadimide compounds in Comparative Examples 1 and 2, and provides good insulating performance. Furthermore, the results of the bending test and the TMA measurements have verified that the bisalkenyl-substituted nadimide A is soft and provides good flexibility, compared with the bisalkenyl-substituted nadimides in Comparative Example 1 and Comparative Example 2.

### [Production of cured product of bisalkenyl-substituted nadimide A and bismaleimide composition]

### (Example 3 to Example 5)

A cured product was produced, using a composition obtained by mixing, at a predetermined ratio, the bisalkenyl-substituted nadimide A synthesized in Example 1 and a bismaleimide BMI-689 purchased from DESIGNER MOLECULES Inc. The respective mixing ratios (parts by mass) are shown in Table 2.

A typical structure of BMI-689 is shown by the following Formula (12).

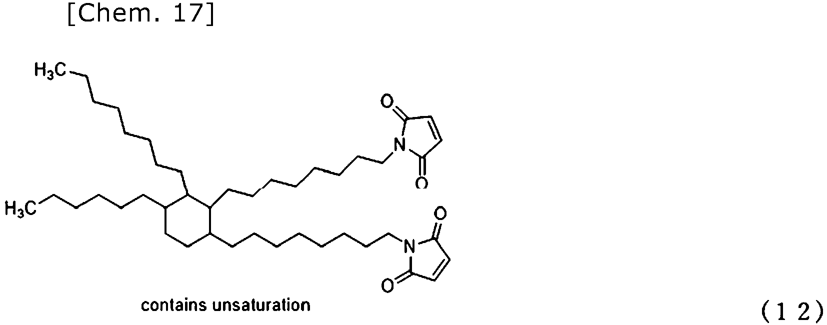

The composition of the bisalkenyl-substituted nadimide A and BMI-689 was placed in a reduced-pressure oven at 170°C to undergo a defoaming treatment, but exhibited almost no generation of voids, and thus, the treatment was performed only for several minutes. Such a long-time defoaming treatment as in Example 1 (the bisalkenyl-substituted nadimide A) was unnecessary.

Next, the composition was poured into a casting mold that was fixed with clips, using two release-agent-coated glass plates and a silicone rubber 2 mm in thickness as a spacer. The resultant object was preliminarily heated at normal pressure at 180°C, and, in the same manner as above, no generation of voids was observed. In addition, the curing of the resin was observed at 180°C. The cured resin was transferred to the electric oven at 200°C under a nitrogen gas stream, heated stepwise up to 240°C, and then retained as it was for 24 hours. Then, the resultant product was cooled approximately to room temperature in the oven, and then, the resultant cured product was taken out of the mold.

### (Comparative Example 3: Production of cured product of bismaleimide BMI-689 alone)

Using the bismaleimide BMI-689, a cured product was produced in the same manner as in Example 3. In the same manner as in Examples 3 to 5, BMI-689 also exhibited no generation of voids, but was not cured at 180°C. Then, BMI-689 was transferred to the electric oven at 200°C under a nitrogen gas stream, heated stepwise up to 240°C, and then retained as it was for 24 hours. Then, the resultant product was cooled to approximately room temperature in the oven, and then, the resultant cured product was taken out of the mold.

The low-temperature curability and the generation of voids as well as the properties of the cured product at 240°C in Examples 3 to 5 and Comparative Example 3 are shown together in Table 2.
In regard to the low-temperature curability, the composition cured by the above-described preliminary heating at 180°C is described as "∘", and the composition not cured is described as "×''. In regard to the generation of voids, the composition in which the generation of voids by the above-described preliminary heating at 180°C was observed is described as "×", and the composition in which no generation of voids was observed is described as "o".

**[Table 2]**

| Measurement and T est | Evaluation Item | Unit | Example 3 | Example 4 | Example 5 | Comparative Example 3 |
|---|---|---|---|---|---|---|
| Mixing ratio | Nadimide A in Example 1 | % by mass | 50 | 30 | 20 | 0 |
| | BMI-689 | % by mass | 50 | 70 | 80 | 100 |
| Low-temperature curability (180 °C) | | - | ○ | ○ | ○ | × |
| Generation of voids | | - | ○ | ○ | ○ | ○ |
| Measurement of dielectric constant and dielectric loss factor | Dielectric constant (10 GHz) | - | 2.20 | 2.27 | 2.25 | 2.24 |
| | Dielectric loss factor (10 GHz) | - | 1.93 × 10⁻³ | 1.72 × 10⁻³ | 1.67 × 10⁻³ | 1.78 × 10⁻³ |
| TMA test | Coefficient of linear expansion | **°C** -1 | 18.8 × 10⁻⁵ | 24.6 × 10⁻⁵ | 22.8 × 10⁻⁵ | 20.5 × 10⁻⁵ |
| Bending test | Maximum stress | MPa | 4.04 | 30.3 | 35.5 | 31.0 |

It has been verified that the compositions (Examples 3 to 5) obtained by mixing the bisalkenyl-substituted nadimide A according to the present invention and the bismaleimide BMI-689 at a specific ratio were successfully cured at a lower temperature than the bisalkenyl-substituted nadimide A alone (Example 2) and BMI-689 alone (Comparative Example 3). In addition, Examples 3 to 5 verified no generation of voids by the preliminary heating performed at 180°C for evaluation of low-temperature curability, and allowed omission of the treatment in which a long-time preliminary heating was performed for defoaming under reduced pressure.

Furthermore, the composition (Example 3) having A and BMI-689 at a mass ratio of 50:50 yielded a very soft cured product having an extremely small maximum stress. The test piece was not fractured but restored to its former state even when repeatedly bent 180° a plurality of times.

In accordance with these results, a bisalkenyl-substituted nadimide according to the present invention can be suitably used as a material for an insulating film to be used in the high-frequency band. Furthermore, blending with another compound such as bismaleimide allows inhibition of generation of voids during curing, allows curing at a lower temperature, and makes it possible to provide a curable resin composition having excellent workability with the insulating performance unchanged.

## Claims

1. A bisalkenyl-substituted nadimide of General Formula (1): 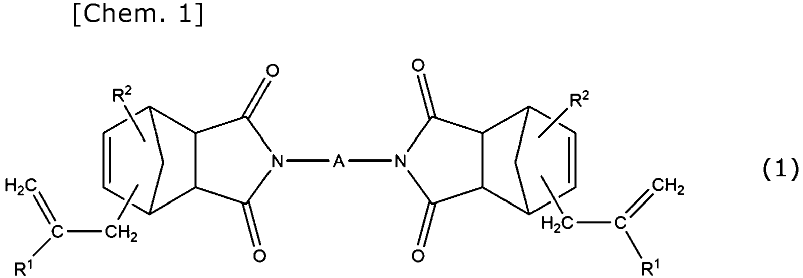
wherein, in Formula (1), R¹ and R² are each a hydrogen atom or a C₁-C₆ alkyl group selected independently; and
A is a divalent hydrocarbon group derived from a dimer acid skeleton.

2. The bisalkenyl-substituted nadimide according to claim 1, wherein the divalent hydrocarbon group derived from the dimer acid skeleton comprises a structure of General Formula (2): 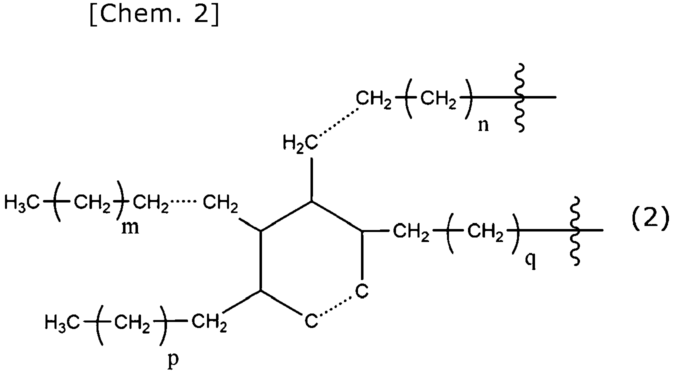 wherein, in Formula (2), m, n, p, and q are each an integer of 1 or greater selected so as to satisfy m + n = 5 to 16 and p + q = 8 to 19; and the bonds shown by the dashed lines are each independently a carbon-carbon single bond or a carbon-carbon double bond, with the proviso that, in a case where the bond shown by each dashed line is a carbon-carbon double bond, Formula (2) is a structure in which the number of hydrogen atoms bound to each of the carbon atoms constituting the carbon-carbon double bond is determined by subtracting 1 from each number shown by Formula (2); and the wavy lines are each a binding site to the nitrogen atom of an imide.

3. A method of producing the bisalkenyl-substituted nadimide according to claim 1 or 2, comprising subjecting a dimer diamine and a compound of General Formula (3) to an addition-dehydration reaction; 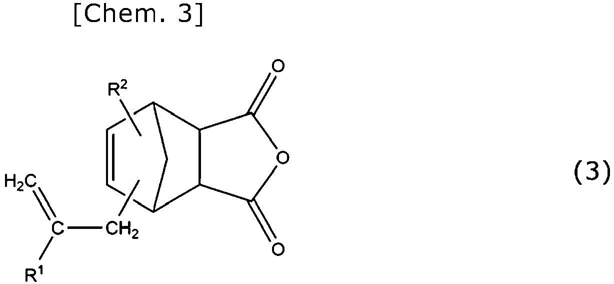 wherein, in Formula (3), R¹ and R² are each a hydrogen atom or a C₁-C₆ alkyl group selected independently.

4. The method of producing the alkenyl-substituted nadimide according to claim 3, wherein the dimer diamine comprises a structure of General Formula (4): 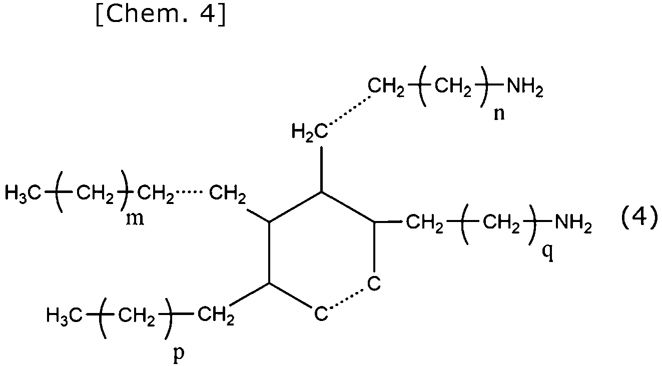 wherein, in Formula (4), m, n, p, and q are each an integer of 1 or greater selected so as to satisfy m + n = 5 to 16 and p + q = 8 to 19; and the bonds shown by the dashed lines are each independently a carbon-carbon single bond or a carbon-carbon double bond, with the proviso that, in a case where the bond shown by each dashed line is a carbon-carbon double bond, Formula (4) is a structure in which the number of hydrogen atoms bound to each of the carbon atoms constituting the carbon-carbon double bond is determined by subtracting 1 from each number shown by Formula (4).

5. A curable material comprising the bisalkenyl-substituted nadimide according to claim 1 or 2.

6. A curable resin composition comprising:
(A) the bisalkenyl-substituted nadimide according to claim 1 or 2; and
(B) at least one compound selected from the group consisting of a maleimide compound, an alkenyl-substituted nadimide (excluding the bisalkenyl-substituted nadimide according to claim 1 or 2), an epoxy resin, a phenol resin, a vinylbenzyl compound, a vinyl compound (excluding a vinylbenzyl compound), a cyclic olefin, a conjugated diene resin having a functional group, and an unsaturated polyester resin.

7. The curable resin composition according to claim 6, wherein the maleimide compound is a bismaleimide of General Formula (5): 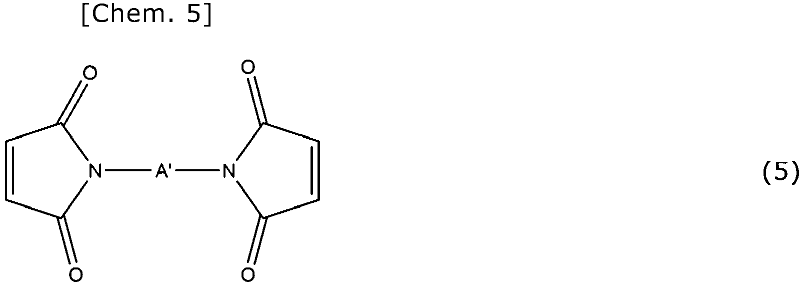 wherein, in Formula (5), A' is a divalent hydrocarbon group.

8. The curable resin composition according to claim 7, wherein A' in the General Formula (5) is a divalent hydrocarbon group derived from a dimer acid skeleton.

9. The curable resin composition according to any one of claims 6 to 8, wherein the mass ratio (A)/(B) of the (A) bisalkenyl-substituted nadimide according to claim 1 or 2 to the (B) is in the range of from 90/10 to 10/90.

10. The curable material according to claim 5, used for an electronic material.

11. The curable resin composition according to any one of claims 6 to 9, used for an electronic material.

12. A cured product obtained by curing the curable material according to claim 5 or 10.

13. A cured product obtained by curing the curable resin composition according to any one of claims 6 to 9 and 11.
